Europäisches Patentamt

European Patent Office

Office européen des brevets

(19)

(11) Veröffentlichungsnummer : **0 011 116**
**B1**

# (12) EUROPÄISCHE PATENTSCHRIFT

(45) Veröffentlichungstag der Patentschrift :
03.02.82

(21) Anmeldenummer : 79103729.4

(22) Anmeldetag : 01.10.79

(51) Int. Cl.³ : **C 07 C 93/193, C 07 C103/70**

(54) **Verfahren zur Herstellung eines flüssigen Gemisches aus Acrylamid und quaternierten Aminoalkylestern oder Aminoalkylamiden der Acryl- oder Methacrylsäure.**

(30) Priorität : 09.11.78 DE 2848627

(43) Veröffentlichungstag der Anmeldung :
28.05.80 (Patentblatt 80/11)

(45) Bekanntmachung des Hinweises auf die Patenterteilung : 03.02.82 Patentblatt 82/05

(84) Benannte Vertragsstaaten :
BE FR GB IT NL

(56) Entgegenhaltungen :
AU - B - 500 009
DE - A1 - 2 608 868
US - A - 4 011 259
Chemical Abstracts, Band 88, Nr. 19, 8. Mai 1978,
Columbus, Ohio, USA, M. Kametani et al. :
« Unsaturated quaternary ammonium salts »
Seite 492, Spalte 1, Abstract Nr. 136125 u

Chemical Abstracts, Band 88, Nr. 19, 8. Mai 1978,
Columbus, Ohio, USA, M. Kametani ammonium
salts » Seite 492, Spalte 1, Abstract Nr. 136126 v

(73) Patentinhaber : Röhm GmbH
Kirschenallee
D-6100 Darmstadt (DE)

(72) Erfinder : Arndt, Peter Joseph, Dr.
Im Säbchen 9
D-6104 Seeheim-Jugenheim 2 (DE)
Erfinder : Lowitz, Joachim
Hauptstrasse 19
D-6146 Alsbach (DE)
Erfinder : Wenzel, Franz, Dr.
Zeyherweg 5
D-6100 Darmstadt (DE)

Imprimerie Jouve, 18, rue St-Denis, 75001 Paris, France

Verfahren zur Herstellung eines flüssigen Gemisches aus Acrylamid und quaternierten
Aminoalkylestern oder Aminoalkylamiden der Acryl- oder Methacrylsäure

Die Quaternierungsprodukte von tertiären Aminoalkylestern oder Aminoalkylamiden der Acryl-oder Methacrylsäure sind wertvolle Ausgangsstoffe für die Herstellung wasserlöslicher Polymerisate, z.B. von Mischpolymerisaten mit Acrylamid. Die tertiären Aminoalkylester und tertiären Aminoalkylamide der Acryl- und Methacrylsäure sind Flüssigkeiten, während ihre Quaternierungsprodukte in reiner Form feste salzartige Verbindungen sind. Es ist im technischen Maßstab nicht in befriedigender Weise möglich, die Quaternierung ohne Zusatz von Lösungs- oder Verdünnungsmitteln durchzuführen, weil sich das Reaktionsgemisch allmählich verfestigt. Unter Zusatz von organischen Lösungsmitteln, wie z.B. Aceton, läßt sich das Quaternierungsgemisch bis zum Abschluß der Quaternierung in einem rührfähigen Zustand halten, jedoch stellt die Abtrennung des Lösungsmittels einen zusätzlichen Arbeitsgang dar. Wenn man die Quaternierung in hochkonzentrierter wäßriger Lösung durchführt, bleibt das Umsetzungsprodukt flüssig und läßt sich in ein festes Polymergel überführen. Die Anwesenheit von Wasser bei der Quaternierung und der anschließenden Polymerisation bewirkt allerdings eine teilweise Hydrolyse der Ester-oder Amidgruppen sowie gegebenenfalls des Alkylierungsmittels, so daß das Quaternierungsprodukt durch die Hydrolyseprodukte des Alkylierungsmittels und durch freie Acryl- oder Methacrylsäure bzw. das Polymerisat durch Einheiten dieser Säuren verunreinigt sein können.

Aus der AU-B 500 009 sowie den in Chemical Abstracts, Band 88, Ref.-Nr. 136125 und 136126 referierten japanischen Patentanmeldungen sind schon Verfahren bekannt, bei denen Aminoalkylester der Acryl- oder Methacrylsäure in organischen Lösungsmitteln, in denen das Quaternierungsprodukt unlöslich ist, quaterniert werden. Als organische Flüssigkeiten werden Ketone, aliphatische und aromatische Kohlenwasserstoffe und Nitrile genannt. Diese Verfahren haben die oben bereits beschriebenen Nachteile, weil in jedem Falle erst das kristallisierte Quaternierungsprodukt isoliert werden muß. Das gilt auch für den Fall der Verwendung von Acrylnitril als flüssiges Medium.

Nach dem Beispiel 8 der US-A 4 011 259 wird durch Umsetzung von Brommethylmethacrylat mit Dimethylhexadecylamin in Acrylnitril als Reaktionsmedium ein quartärer Ammoniumalkylester der Methacrylsäure hergestellt. Das Reaktionsprodukt bleibt gelöst und läßt sich unmittelbar mit dem Acrylnitril und gegebenenfalls weiteren Comonomeren in ein Mischpolymerisat unwandeln. Dieses Verfahren ist nicht auf die Quaternierung von Dimethylaminoäthylmethacrylat mit Methylchlorid übertragbar, weil das Quaternierungsprodukt, wie oben schon erwähnt, in kristalliner Form ausfällt.

In der DE-A 2 608 868 wird die Behauptung aufgestellt, daß die Quaternierung von Aminoalkylestern in hochkonzentrierter wäßriger Lösung nur in geringem Maße oder gar nicht zu Hydrolyse führe. Das ist aber nicht der Fall; vielmehr entstehen selbst bei niedrigem Wassergehalt des Gemisches erhebliche Anteile an Hydrolyseprodukten.

Der Erfindung liegt die Aufgabe zugrunde, tertiäre Aminoalkylester oder tertiäre Aminoalkylamide der Acryl- oder Methacrylsäure in Abwesenheit von Wasser in der Weise zu quaternieren, daß während der gesamten Dauer der Umsetzung ein rührfähiges System vorliegt und daß ein Reaktionsprodukt entsteht, das sich ohne zusätzliche Reinigungs- und Aufarbeitungsverfahren unmittelbar zur Polymerisation einsetzen läßt.

Die Aufgabe wird erfindungsgemäß durch das Verfahren gemäß Anspruch 1 gelöst. Man unterwirft den tertiären Aminoalkylester oder das tertiäre Aminoalkylamid im Gemisch mit Acrylamid der Einwirkung des Alkylierungsmittels. Acrylamid ist bei Raumtemperatur ein Feststoff, der in dem flüssigen Aminoalkylester oder Aminoalkylamid nur geringfügig löslich ist. Im Laufe der Umsetzung bildet sich aus dem Acrylamid und dem Quaternierungsprodukt eine flüssige Phase, in der ein vollständiger Umsatz des Ausgangsmaterials erreicht werden kann. Die Umsetzung verläuft rasch und ohne Nebenreaktionen; infolge der Abwesenheit von Wasser ist eine Hydrolyse ausgeschlossen. Der Anteil des Acrylamids in dem Gemisch mit dem zu alkylierenden Ausgangsstoff beträgt 30 bis 80 Gew.-%.

Das Reaktionsprodukt ist ein Gemisch aus dem salzartigen Quaternierungsprodukt und Acrylamid. Es läßt sich unmittelbar in entsprechende Mischpolymerisate überführen. Die Erfindung hat daher ihre größte Bedeutung als Teil eines Herstellungsverfahrens von derartigen Mischpolymerisaten.

Die bevorzugten Ausgangsstoffe sind N,N-Dialkylaminoalkylester und N,N-Dialkylaminoalkylamide der Acryl- und Methacrylsäure, wozu auch solche Verbindungen zu rechnen sind, in denen die N-ständigen Alkylreste zu einem Piperidino-, Morpholino- oder Piperazinring verbunden sind. Besonders bevorzugte Ausgangsstoffe sind N,N-Dialkylaminoalkylester der Acryl- und Methacrylsäure, in denen die N-Alkylreste jeweils 1 bis 4 und die zwischen dem Aminostickstoffatom und dem Estersauerstoffatom bzw. Amidostickstoffatom stehende Alkylgruppe 2 bis 4 Kohlenstoffatome enthält. Die Ester sind wiederum vor den Amiden bevorzugt. Beispiele für diese Ester sind Dimethylaminoäthylester, Diäthylaminoäthylester, 2-Dimethylaminopropylester, Morpholinoäthylester, N-Butyl-N-Methylaminoäthylester und Dibutylaminoäthylester der Acryl- oder Methacrylsäure. Als Beispiele für Ausgangsstoffe mit Amidstruktur seien Dimethylaminopropylacrylamid und -methacrylamid besonders hervorgehoben.

Als Alkylierungsmittel werden bevorzugt Alkylhalogenide, insbesondere Alkylchloride oder Dialkylsulfate eingesetzt, die 1 bis 4 C-Atome im Alkylrest enthalten. Methylchlorid und Dimethylsulfat sind besonders bevorzugt. Sie werden in wenigstens stöchiometrischer Menge eingesetzt. Methylchlorid hat den besonderen Vorzug, daß sich ein nicht umgesetzter Überschuß leicht über die Gasphase entfernen läßt.

Die Umsetzung kann kontinuierlich oder diskontinuierlich in einfachen Rührgefäßen durchgeführt werden, die zweckmäßigerweise geschlossen sind und getrennte Einleitungsanschlüsse für Acrylamid, den zu quaternierenden Ausgangsstoff und das Alkylierungsmittel haben. In der Regel wird zur Unterdrückung einer vorzeitigen Polymerisation während der Quaternierung ein Polymerisationsinhibitor, wie Hydrochinonmonomethyläther, zugegeben. Man kann gegebenenfalls vor, während oder nach der Quaternierungsreaktion weitere radikalisch polymerisierbare Monomere, die gegenüber dem Alkylierungsmittel inert sind, zusetzen, um das Gemisch anschließend z.B. in ein Terpolymerisat überzuführen. Dafür kommen besonders wasserlösliche Monomere, wie Methacrylamid, Vinylpyrrolidon, Hydroxyalkylester der Acryl- oder Methacrylsäure, in begrenztem Umfang auch wasserunlösliche Monomere in Betracht, sofern sie mit dem Reaktionsprodukt mischbar sind.

Die Umsetzung wird bei Temperaturen über 30 °C, vorzugsweise im Bereich von 35 bis 100 °C durchgeführt. Auf jeden Fall muß die Schmelztemperatur des Gemisches überschritten werden, was insbesondere bei niedrigem Gehalt an Acrylamid eine Umsetzungstemperatur von etwa 50 °C erforderlich machen kann. Um die Polymerisationsgefahr möglichst gering zu halten, sollte die Temperatur nicht höher als zur Aufrechterhaltung der Homogenität nötig gewählt werden. Bei der Alkylierung mit gasförmigen Alkylierungsmitteln, wie Methylchlorid, arbeitet man zweckmäßig im Druckgefäß unter 1 bis 5 bar. Nach einer Reaktionsdauer von 15 bis 60 min ist in der Regel ein vollständiger Umsatz erreicht.

Das flüssige Reaktionsprodukt wird vorzugsweise unmittelbar nach der Herstellung polymerisiert. Zu diesem Zweck kann ein üblicher radikalbildender Initiator, z.B. Ammoniumpersulfat, Wasserstoffperoxid, Azo-bis-cyanvaleriansäure oder ein Redox-System, zu dem flüssigen Reaktionsprodukt zugegeben und dieses in dünner Schicht, z.B. auf einem bewegten Band, in bekannter Weise polymerisiert werden. Der Zusatz geringer Mengen Wasser vor Beginn der Polymerisation fördert die Homogenität des polymerisierten Endproduktes. Mann kann jedoch auch eine wäßrige Lösung beliebiger Konzentrationen herstellen und diese nach bekannten Verfahren polymerisieren.

Die entstehenden kationischen Acrylamid-Mischpolymerisate sind wertvolle Hilfsmittel für die Klärung von Abwässern und Schlammsuspensionen, Retentionsmittel für die Papierherstellung und Verdickungsmittel für wäßrige Flüssigkeiten.

Beispiel 1

3,5 kg Dimethylaminoäthylmethacrylat und 10,85 kg Acrylamid werden in einem 20 l-Druck-Kessel aus VA-Stahl unter Rühren gemischt. In den geschlossenen Kessel wird Methylchlorid eingeleitet, wobei die Temperatur rasch ansteigt und durch äußere Kühlung bei 45-50 °C gehalten wird. Der Methylchlorid-Partialdruck wird auf 3 bis 3,5 bar eingestellt. Nach 50 min ist die Quaternierung mit einem Umsatz von über 99 % beendet. Das Produkt ist flüssig und enthält geringe kristalline Anteile, die sich nach Zusatz von etwas Wasser auflösen.

Beispiel 2

Das Verfahren gemäß Beispiel 1 wird mit einem Gemisch aus 8,2 kg Dimethylaminoäthylmethacrylat und 7,2 kg Acrylamid wiederholt. Der Methylchloridpartialdruck wird auf 1,5 bis 3,5 bar eingestellt und die Temperatur durch Kühlung bei 50 °C gehalten. Nach 60 min ist ein Umsatz von 99,5 % erreicht. Das Endprodukt ist bei 50 °C flüssig und klar.

Beispiel 3

2,75 kg Dimethylaminoäthylacrylat und 8,6 kg Acrylamid werden, wie in Beispiel 1 beschrieben, gemischt und bei 50 °C mit Methylchlorid quaterniert. Nach 60 min ist in 99,5 % Ausbeute das Quaternierungsprodukt als klare Schmelze entstanden.

Beispiel 4

3,4 kg Dimethylaminopropylmethacrylamid und 4,4 kg Acrylamid werden, wie in Beispiel 1 beschrieben, gemischt und bei 50 °C mit Methylchlorid unter einem Partialdruck von 1 bis 2 bar quaterniert. Nach 90 min wird in 99 %iger Ausbeute das Quaternierungsprodukt bei 50 °C als zähflüssige, klare Schmelze gewonnen.

Beispiel 5

2,0 kg Diäthylaminoäthylacrylat und 2,6 kg Acrylamid werden, wie in Beispiel 1 beschrieben, gemischt und bei 60 °C mit Methylchlorid während 3 Std. mit einem Umsatz von 97 % quaterniert. Es entsteht eine bei 50 °C klare Schmelze.

Beispiel 6

4,6 kg Diäthylaminoäthylacrylat und 7,8 kg Acrylamid werden in einem geschlossenen, kühlbaren 20 l-VA-Stahl-Kessel gemischt und 3,4 kg Dimethylsulfat langsam zugesetzt, wobei man die Reaktionstemperatur nicht über 25 °C ansteigen läßt. Nach 60 min ist die Quaternierung beendet.

Die auf 50 °C erwärmte Schmelze vom pH-Wert 8,0 ist dünnflüssig und klar und kann zur Polymerisation eingesetzt werden.

**Ansprüche**

1. Verfahren zur Herstellung eines flüssigen, unmittelbar polymerisierbaren Gemisches aus Acrylamid und Quaternierungsprodukten von tertiären Aminoalkylestern oder tertiären Aminoalkylamiden der Acryl- oder Methacrylsäure durch Umsetzen der entsprechenden Ester bzw. Amide mit einem Alkylierungsmittel in Abwesenheit von Wasser, dadurch gekennzeichnet, daß man ein Gemisch aus dem tertiären Aminoalkylester oder dem tertiären Aminoalkylamid und Acrylamid, worin das Acrylamid 30 bis 80 Gew.-% des Gemisches ausmacht, oberhalb 30 °C und oberhalb der Schmelztemperatur des Gemisches der Einwirkung des Alkylierungsmittels unterwirft.

2. Verfahren nach Anspruch 1, dadurch gekennzeichnet, daß ein N,N-Dialkylaminoalkylester oder ein N,N-Dialkylaminoalkylamid der Acryl- oder Methacrylsäure mit einem Alkylierungsmittel quaterniert wird.

3. Verfahren nach Anspruch 2, dadurch gekennzeichnet, daß ein N,N-Dialkylaminoalkylester oder ein N,N-Dialkylaminoalkylamid der Acryl- oder Methacrylsäure mit 1 bis 4 C-Atomen in jedem N-Alkylrest und 2 bis 4 C-Atomen in der Alkylester- bzw. Alkylamidogruppe quaterniert wird.

4. Verfahren nach den Ansprüchen 1 bis 3, dadurch gekennzeichnet, daß das Alkylierungsmittel ein Alkylchlorid oder Dialkylsulfat mit 1 bis 4 C-Atomen im Alkylrest ist.

**Claims**

1. Process for preparing a liquid, directly polymerisable mixture of acrylamide and quaternisation products of tertiary aminoalkyl esters or tertiary aminoalkylamides of acrylic or methacrylic acid by reacting the corresponding esters or amides with an alkylating agent in the absence of water, characterised in that a mixture of the tertiary aminoalkyl ester or the tertiary aminoalkyl amide and acrylamide, wherein the acrylamide constitutes 30 to 80 % by weight of the mixture, is subjected to the action of the alkylating agent at above 30 °C and above the melting temperature of the mixture.

2. Process according to claim 1, characterised in that an N,N-dialkylaminoalkyl ester or an N,N-dialkylaminoalkyl amide of acrylic or methacrylic acid is quaternised with an alkylating agent.

3. Process according to claim 1, characterised in that an N,N-dialkylaminoalkyl ester or an N,N-dialkylaminoalkylamide of acrylic or methacrylic acid with 1 to 4 carbon atoms in each N-alkyl group and 2 to 4 carbon atoms in the alkyl ester or alkylamido group is quaternised.

4. Process according to claims 1 to 3, characterised in that the alkylating agent is an alkyl chloride or dialkyl sulphate with 1 to 4 carbon atoms in the alkyl group.

**Revendications**

1. Procédé de préparation d'un mélange liquide, directement polymérisable, d'acrylamide et de produits de quaternisation d'esters aminoalkyliques tertiaires ou d'aminoalkylamides tertiaires de l'acide acrylique ou méthacrylique par réaction des esters ou amides correspondant avec un agent alkylant en l'absence d'eau, caractérisé en ce que l'on soumet un mélange de l'ester aminoalkylique tertiaire ou de l'aminoalkylamide tertiaire et d'acrylamide dans lequel l'acrylamide représente de 30 à 80 % du poids du mélange, à l'action de l'agent alkylant à une température supérieure à 30 °C et supérieure à la température de fusion du mélange.

2. Procédé selon la revendication 1, caractérisée en ce que l'on quaternise par un agent alkylant un ester N,N-dialkylaminoalkylique ou N,N-dialkylaminoalkylamide de l'acide acrylique ou méthacrylique.

3. Procédé selon la revendication 2, caractérisé en ce que l'on quaternise un ester N,N-dialkylaminoalkylique ou N,N-dialkylaminoalkylamide de l'acide acrylique ou méthacrylique contenant de 1 à 4 atomes de carbone dans chaque groupe N-alkyle et 2 à 4 atomes de carbone dans le groupe ester alkylique ou alkylamido.

4. Procédé selon les revendications 1 à 3, caractérisé en ce que l'agent alkylant est un chlorure d'alkyle ou un sulfate de dialkyle contenant de 1 à 4 atomes de carbone par groupe alkyle.